(19) Europäisches Patentamt
European Patent Office
Office européen des brevets

(11) **EP 2 196 140 B1**

(12) # EUROPÄISCHE PATENTSCHRIFT

(45) Veröffentlichungstag und Bekanntmachung des Hinweises auf die Patenterteilung:
**19.03.2014 Patentblatt 2014/12**

(51) Int Cl.:
*A61B 5/00* *(2006.01)*    *A61B 5/053* *(2006.01)*
*G01N 33/487* *(2006.01)*

(21) Anmeldenummer: **09015351.1**

(22) Anmeldetag: **11.12.2009**

(54) **Verfahren und Einrichtung zur nicht invasiven Bestimmung des Blutzuckergehalts im Blut**

Method and device for non-invasive determination of the blood sugar level

Procédé et dispositif de détermination non invasive du taux de glycémie dans le sang

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **11.12.2008 DE 102008061900**
**07.12.2009 EP 09015125**

(43) Veröffentlichungstag der Anmeldung:
**16.06.2010 Patentblatt 2010/24**

(73) Patentinhaber: **Trout GmbH**
**34119 Kassel (DE)**

(72) Erfinder:
- **Thon, Susanne**
  **34134 Kassel (DE)**
- **Fischer, Hartmut**
  **34125 Kassel (DE)**
- **Völkel, Andreas, Dr.**
  **34277 Fuldabrück (DE)**

- **Bussas, Martin**
  **34119 Kassel (DE)**

(74) Vertreter: **Walther, Walther & Hinz GbR**
**Heimradstrasse 2**
**34130 Kassel (DE)**

(56) Entgegenhaltungen:
**WO-A1-00/09996    WO-A1-99/39627**
**WO-A1-02/069798    WO-A2-2008/141306**
**US-B2- 6 841 389**

- **CHEOL-TAEK KIM ET AL: "Variable Projection Method and Levenberg-Marquardt Algorithm for Neural Network Training", IEEE INDUSTRIAL ELECTRONICS, IECON 2006 - 32ND ANNUAL CONFERENCE ON, IEEE, PISCATAWAY, NJ, USA, 1 November 2006 (2006-11-01), pages 4492-4497, XP031077235, ISBN: 978-1-4244-0135-2**

**Beschreibung**

Die vorliegende Erfindung betrifft ein Verfahren und eine Einrichtung zur nicht invasiven Bestimmung des Blutzuckergehalts im Blut durch Messung der Impedanz des Körpers oder eines Teils des Körpers eines Patienten.

Die Messung des Blutzuckergehalts ist für Diabetiker von immanenter Wichtigkeit. Schlussendlich dient die Kenntnis über den momentanen Blutzuckergehalt zur Entscheidungsfindung darüber, ob und gegebenenfalls wie viel Insulin sich der Diabetiker zuführen muss. Eine solche Kontrolle hat mehrfach über den Tag verteilt zu erfolgen, um sicherzustellen, dass größere Sprünge des Zuckergehalts im Blut vermieden werden. Üblicherweise erfolgt eine vier- bis sechsmalige Kontrolle des Blutzuckergehalts über einen Zeitraum von 24 Stunden.

Die Überprüfung des Blutzuckergehalts mit Hilfe sogenannter Blutzuckermessstreifen ist bekannt. Hierbei wird üblicherweise so vorgegangen, dass sich die Patienten mit einer Nadel in die Fingerkuppe stechen, wobei das austretende Blut auf den Blutzuckermessstreifen gegeben wird, welcher anhand einer Verfärbung anzeigt, wie hoch in etwa der Blutzuckergehalt tatsächlich in dem Moment ist. Diese Methode hat den Nachteil, dass sie relativ ungenau ist. Der Genauigkeitsgrad einer solchen Messung liegt zwischen +/- 15 und 20 %. Darüber hinaus ist nachteilig, dass durch das vielfach wiederholte Stechen - wie bereits ausgeführt bis zu sechsmal am Tag - mit einer Nadel in die Fingerkuppe dies auf Dauer zu einer Verhärtung des Gewebes in der Fingerkuppe führt. Des Weiteren besteht die Gefahr von Infektionen. Nicht zuletzt ist die Überprüfung des Blutzuckergehalts mit Messstreifen teuer, da der Preis für solche Messstreifen zwischen € 1,00 und € 2,00 pro Stück beträgt.

Es besteht daher ein lebhaftes Interesse an alternativen Methoden zur Bestimmung des Blutzuckergehalts. Insbesondere von Interesse sind hierbei sogenannte nicht invasive Methoden zur Ermittlung des Blutzuckergehalts. Aus der US 6,841,389 B2 ist in diesem Zusammenhang ein nicht invasives Verfahren zur Bestimmung des Blutzuckergehalts bekannt. Dieses Verfahren nutzt lineare Näherungsfunktionen 1.

Ableitung für die Beziehung zwischen Blutzuckergehalt und Widerstand der Haut.

Das Berechnungsmodul berücksichtigt eine Änderung im Blutzuckergehalt. Die Koeffizienten in der Nährungsfunktion werden zu Beginn durch Messung des Blutzuckergehalts auf invasivem Weg bestimmt. Allerdings hat sich herausgestellt, dass die in der US-Schrift angegebenen Berechnungsmethoden zu keinen hinreichend genauen Ergebnissen bei der Bestimmung des Blutzuckerwertes führen. Dies liegt im Wesentlichen darin begründet, dass die mathematischen Modelle starr sind.

Aus WO 02/069798 A1 ist in diesem Zusammenhang ein nicht invasives Verfahren zur Bestimmung des Blutzuckergehaltes bekannt, bei dem das Verfahren auf mindestens zwei Eingangsgrößen zurückgreift, nämlich die Hautimpedanz und/oder die Herzfrequenz und/oder QT Intervall (EKG) und/oder die Alphawellen aus dem EEG. Auf der Grundlage von zwei oder mehr der in solcherweise gewonnenen Daten erfolgt die Ermittlung von Tendenzen zur Bestimmung einer Unter- oder Überzuckerung. Der Blutzuckerwert als solcher kann hiermit nicht bestimmt werden.

Aus der EP 1 309 271 1 ist ein Verfahren bekannt, das die Möglichkeit der Warnung vor hypoglykämischen Erzeugnissen vorsieht. Aus dem gemäß dieser EP-Schrift bekannten Verfahren wird so vorgegangen, dass kontinuierlich eine Probenentnahme beim Patienten stattfindet, um den Glukosegehalt zu bestimmen. Dieses System ist auch unter dem Stichwort der Methode der reversen Iontophorese (Elektroosmose) bekannt. Das heißt, der Haut werden kontinuierlich Zellen (Gewebeflüssigkeit) entnommen, um hieraus den Glukosewert zu bestimmen. Zur Auswertung kommt auch kein künstliches neuronales Netz zur Anwendung. Die Art der Auswertung erfolgt hierbei offensichtlich lediglich durch die sogenannte TSES Funktion sowie einem intelligenten Datenverarbeitungsalgorithmus. Der aktuelle Blutzuckerwert kann somit hiermit nicht bestimmt werden.

Aus der WO 2008/141306 A2 ist bekannt mittels nichtlinearer Gleichungen einen Zusammenhang zwischen bekannten physiologischen und den physikalischen Parametern herzustellen, wie Blutzuckergehalt und an der Haut gemessenen Impedanzwerten sowie der Körpertemperatur. Die in solcher Weise ermittelten Gleichungen können dann dazu dienen mit bekannten Impedanzwerten und Werten für die Körpertemperatur den aktuellen Blutzuckergehalt zu berechnen.

Die der Erfindung zugrunde liegende Aufgabe besteht demzufolge darin, ein Verfahren der eingangs genannten Art bereitzustellen, das mit hinreichender Genauigkeit eine Bestimmung des Blutzuckergehaltes zulässt, wobei schlussendlich lediglich die Impedanz der Haut des Patienten Berücksichtigung findet.

Zur Lösung der Aufgabe wird ein Verfahren der eingangs genannten Art vorgeschlagen, das sich durch folgende Schritte auszeichnet:

- Einbringen eines hoch- und eines niederfrequenten Stroms in den Körper eines Patienten, wobei die Elektroden zur Einbringung des hochund des niederfrequenten Stroms beabstandet zueinander am Körper des Patienten angelegt sind;
- Eingabe der ermittelten HF- und LF-Impedanzen durch eine Messeinrichtung und Eingabe der ermittelten HF- und LF-Impedanzen in ein kalibriertes künstliches, neuronales Netzwerk;
- Generierung einer Mehrzahl von neuronalen Netzen mit unterschiedlichen Startwerten zur genaueren Ermittlung des Blutzuckerwertes aus gemessenen Impedanzen, wobei zum Training des neuronalen Netzwerkes von der Mehrzahl der Netze eine Anzahl von Netzen mit den Blutzuckerwerten herangezogen wird, die zu denen der während des Kalibrierungsschrittes invasiv ermittelten Blutzuckerwerten die geringste Abweichung aufweisen;

- Kalibrierung des künstlichen neuronalen Netzwerkes durch Eingabe mindestens jeweils einer über einen bestimmten Zeitraum gemessenen LF- und HF-Impedanz sowie gegebenenfalls mindestens jeweils eines Temperaturmesswertes in die neuronalen Netze sowie mindestens eines in dem Zeitraum invasiv ermittelten Blutzuckerwertes, wobei der anhand der HF- und LF-Impedanzen und gegebenenfalls der Temperatur durch das künstliche neuronale Netz ermittelte Blutzuckerwert mit dem invasiv gemessenen Blutzuckerwert verglichen und mittels eines numerischen Verfahrens, zum Beispiel mittels des Levenberg-Marquardt-Algorithmus, die Abweichung reduziert wird;

- Ermittlung des Blutzuckergehalts durch Mittelwertbildung der Ausgabewerte der herangezogenen neuronalen Netze

- Anzeige des durch das neuronale Netzwerk ermittelten Blutzuckergehalts auf einer Anzeige.

Der hochfrequente Strom befindet sich hierbei im Bereich von 100-400 KHz, insbesondere von 200 KHz; der niederfrequente Strom liegt im Bereich von 10-100 kHz, insbesondere im Bereich von 20-50 kHz. Die Größe der Frequenz bestimmt, in welcher Tiefe subkutan im Gewebe die Impedanz gemessen wird. In den oben angegebenen Bereichen, so hat sich herausgestellt, lässt sich aus den genaueren Impedanzen ein hinreichend genauer Blutzuckerwert ermitteln. Hierdurch wird auch erreicht, dass der Blutzuckergehalt im Wesentlichen kontinuierlich gemessen wird, also in bestimmten zeitlichen Abständen, z. B. 10 Sekunden. Vorteilhaft hierbei ist, dass der Patient somit in der Lage ist, das Unter- oder Überschreiten von Grenzen, d. h. das Auftreten von Hypoglykämie oder Hyperglykämie zu vermeiden.

Die Erfindung betrifft ebenfalls eine Einrichtung zur nicht invasiven Bestimmung des Blutzuckergehalts im Blut durch Messung der Impedanzen des Körpers des Patienten oder eines Teils des Körpers des Patienten, umfassend zwei beabstandet zueinander am Körper des Patienten anbringbaren Elektroden zur Einbringung eines nieder- und eines hochfrequenten Stroms in den Körper des Patienten, weiterhin umfassend eine Messeinrichtung zur Erfassung der Impedanzen sowie einer Rechnereinheit in Form eines Mikroprozessors zur Verarbeitung der ermittelten HF- und LF-Impedanzen in einem in dem Mikroprozessor installierten kalibrierten neuronalen Netzwerk, wobei zum Training des neuronalen Netzwerkes eine Mehrzahl von neuronalen Netzen mit unterschiedlichen Startwerten zur genaueren Ermittlung des Blutzuckerwertes aus den gemessenen Impedanzen generiert wird, wobei von der Mehrzahl der Netze eine Anzahl von Netzen ausgewählt wird, deren Werte zu denen der während des Kalibrierungsschritts invasiv ermittelten Blutzuckerwerten die geringste Abweichung aufweisen, wobei die Kalibierung des künstlichen neuronalen Netzwerkes durch Eingabe mindestens jeweils einer über einen bestimmten Zeitraum gemessenen LF-und HF-Impedanz sowie gegebenenfalls mindestens jeweils eines Temperaturmesswertes in die neuronalen Netze sowie mindestens eines in dem Zeitraum invasiv ermittelten Blutzuckerwertes erfolgt, wobei der anhand der HF- und LF-Impedanzen und gegebenenfalls der Temperatur durch das künstliche neuronale Netz ermittelte Blutzuckerwert mit dem invasiv gemessenen Blutzuckerwert verglichen und mittels eines numerischen Verfahrens, zum Beispiel mittels des Levenberg-Marquardt-Algorithmus, die Abweichung reduziert wird, wobei der Blutzuckergehalt durch Mittelwertbildung der Ausgabewerte der herangezogenen neuronalen Netze ermittelt wird; weiterhin umfassend eine Anzeigeeinrichtung zur Anzeige des durch das neuronale Netzwerk ermittelten Blutzuckergehaltes.

Die Einrichtung umfasst hierbei zwei Manschetten, die an den Körper angelegt werden und mittels derer der HF- und der LF-Strom in den Körper eingebracht wird. Die jeweilige Impedanz wird durch einen Mikroprozessor mit dem neuronalen Netzwerk verarbeitet. Der Mikroprozessor ist hierbei getrennt von dem LF- bzw. HF-Generator in einem gesonderten Gehäuse untergebracht.

Weitere vorteilhafte Ausgestaltungen und Weiterbildungen ergeben sich aus den Unteransprüchen.

Anhand der Zeichnungen wird die Erfindung nachstehend beispielhaft näher erläutert.

Figur 1    zeigt schematisch den Aufbau des Gewebes;
Figur 2    zeigt schematisch die Funktionsweise eines Neurons;
Figur 3    zeigt schematisch den Aufbau eines künstlichen neuronalen Netzes;
Figur 4    zeigt ein Beispiel für das Ergebnis einer Messung innerhalb eines Zeitraums eines Diabetes (Typ 1) Patienten.

Bekannt ist, dass der Blutzuckergehalt mit der Impedanz des Blutes korreliert. Im Einzelnen gilt in Bezug auf den Zusammenhang zwischen Blutzucker G und der Impedanz Z folgende Beziehung:

$$G = f(Z) : G \uparrow \underline{Osmose}\ V_{out} \uparrow \rightarrow C^{LF} \uparrow \rightarrow Z \downarrow$$

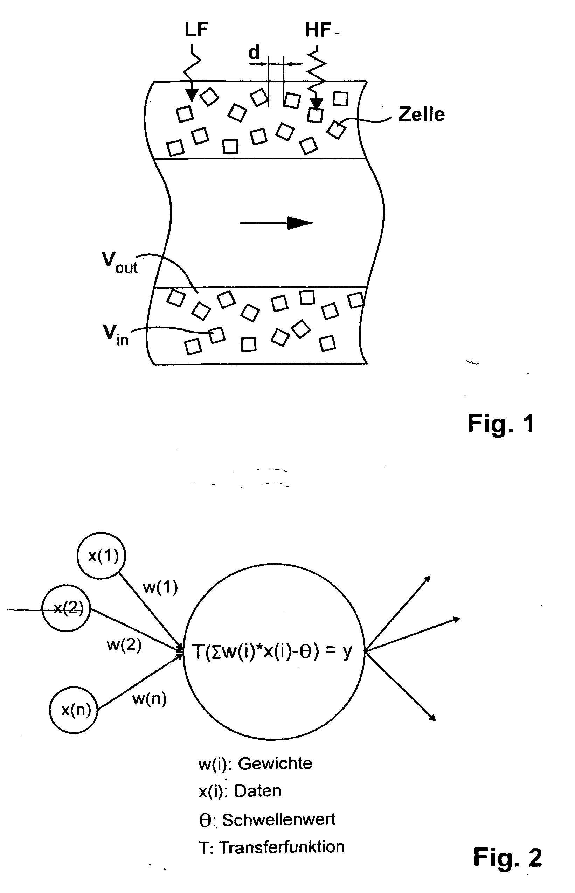

Der Blutzuckerspiegel beeinflusst den osmotischen Druck und damit die Leitfähigkeit des Gewebes. Bei steigendem Blutzuckerspiegel steigt auch der osmotische Druck. Dies hat zur Folge, dass die Zellen kleiner werden, das Flüssigkeitsvolumen $V_{out}$ allerdings größer wird, womit naturgemäß auch der Abstand zwischen den einzelnen Zellen steigt, wodurch die Leitfähigkeit C steigt und die Impedanz Z infolgedessen fällt. Das heißt, in Folge osmotischer Vorgänge steigt bei zunehmendem Blutzuckerspiegel das extrazelluläre Flüssigkeitsvolumen $V_{out}$ an. Anders ausgedrückt bedeutet dies, dass, wenn der Blutzuckerspiegel steigt, der osmotische Druck ebenfalls ansteigt, jedoch aufgrund des ansteigenden Druckes die Zelle kleiner wird. Da die Zelle kleiner wird, wird das extrazelluläre Volumen größer, was zu einer Steigerung der Leitfähigkeit führt, was wiederum zu einem Fallen der Impedanz führt (Fig. 1).

Des Weiteren gilt für den Zusammenhang zwischen Impedanz und Flüssigkeitsvolumen Folgendes:

Durch Einbringung eines hochfrequenten Stroms wird die Impedanz in der Gesamtflüssigkeitsmenge ermittelt. Dies geschieht z. B. durch Anlage zweier Manschetten, die beabstandet zueinander an dem Arm des Patienten angelegt werden. Die Manschetten weisen die Elektroden auf.

Die Gesamtmenge an Flüssigkeit stellt ergibt sich zu $V_{tot} = V_{in} + V_{out}$ ($V_{in}$ = Flüssigkeitsvolumen in der Zelle; $V_{out}$ = Flüssigkeitsvolumen außerhalb der Zelle). Bei Einbringung eines niederfrequenten Stroms in den Körper kann die Impedanz lediglich in dem Flüssigkeitsvolumen außerhalb der Zellen dargestellt werden. Insgesamt ergibt sich für den Zusammenhang zwischen Impedanz Z und dem Flüssigkeitsvolumen im Gewebe folgende Abhängigkeit:

$$Z = f(V) \begin{cases} Z \sim \dfrac{1}{C^{LF}} \sim \dfrac{1}{V_{out}} \\ Z \sim \dfrac{1}{C^{HF}} \sim \dfrac{1}{V_{in}} \end{cases}$$

Das heißt, für den niederfrequenten Anteil verhält sich der Impedanzwert umgekehrt proportional zur niederfrequenten Leitfähigkeit und damit umgekehrt proportional zum außerzellulären Flüssigkeitsvolumen $V_{out}$. Im Fall des hochfrequenten Anteils verhält sich der Impedanzwert ebenfalls umgekehrt proportional zur hochfrequenten Leitfähigkeit C und damit umgekehrt proportional zur Summe $V_{tot}$ aus interzellulärem Flüssigkeitsvolumen $V_{in}$ und außerzellulärem Flüssigkeitsvolumen $V_{out}$.

Vorteilhaft bei der Verwendung eines künstlichen neuronalen Netzwerkes mit mehreren Netzen ist, dass in Abhängigkeit von der Häufigkeit der Messungen und insbesondere vorangegangener Trainingsvorgänge das ganze Verfahren auf Dauer immer genauer wird. Das heißt, das Verfahren stellt sich im Laufe der Zeit immer besser auf den speziellen Patienten ein. Dies deshalb, weil - wie bereits ausgeführt - ein neuronales Netz oder Netzwerk grundsätzlich lernfähig ist.

Eine weitere Erhöhung der Genauigkeit erfährt das Verfahren durch Berücksichtigung der Körpertemperatur. Ein Anstieg des Zuckergehalts im Blut führt zu einer Temperaturverminderung, was einen Abfall der Leitfähigkeit des Gewebes zur Folge hat. Es wurde beispielsweise herausgefunden, dass eine Temperaturdifferenz von 1° C eine Änderung der Impedanz von 2 % zur Folge hat.

Bereits an anderer Stelle wurde erläutert, dass sich bei steigendem Zuckergehalt das extrazelluläre Volumen $V_{out}$ ändert, was eine fallende Impedanz zur Folge hat. In Bezug auf die Temperatur wurde dargelegt, dass mit steigendem Zuckergehalt die Temperatur sinkt, und infolge einer Leitfähigkeitsverminderung die Impedanz für den eingebrachten niederfrequenten Strom steigt. Insgesamt stellt sich der Blutzuckergehalt als Funktion der Temperatur und des extrazellulären Volumens wie folgt dar:

$$G = f(T, V_{out}) : G \uparrow \begin{cases} \Rightarrow T \;\downarrow\; \Rightarrow Z^{LF} \uparrow \\ \Rightarrow V_{out} \uparrow \Rightarrow Z^{HF} \downarrow \end{cases}$$

Das bedeutet aber auch, dass zur Kalibrierung der Mehrzahl von Netzen dem Startwerten mit unterschiedlichen Impedanzen noch die entsprechenden Temperaturwerte hinzugegeben werden.

Im Einzelnen ist vorgesehen, dass die Körpertemperatur am Körpers des Patienten, d. h. auf der Oberfläche der Haut, im Bereich der Elektroden gemessen wird, da Messungen der Temperatur außerhalb des Bereichs des Elektroden zur Einbringung des nieder- und des hochfrequenten Stroms in den Körper des Patienten zur Verfälschung der Relation der Messwerte zueinander führen können. Insofern ist auch vorteilhaft vorgesehen, dass die Körpertemperatur zum Zeitpunkt der Messung der Impedanz ermittelt wird.

Berücksichtigt wird bei der Messung der Impedanz die Feuchtigkeit oder Trockenheit der Haut. Bekannt ist, dass mit zunehmender Trockenheit der Hautoberfläche der Widerstand wächst. Der Kontakteinfluss über die Feuchtigkeit wird nun dadurch kompensiert, dass jeweils ein zweites Elektrodenpaar verwendet wird. Das heißt, die Manschetten, die am Arm des Patienten antigen, besitzen jeweils ein zweites Elektrodenpaar. Die Kompensation bezieht sich für die Elektroden auf den Kontaktwiderstand, der durch die Verwendung eines weiteren Elektrodenpaares kompensiert werden kann. Der Glukoseanstieg führt zu einer höheren Leitfähigkeit des Gewebes, jedoch kann sich die Leitfähigkeit der Elektroden, z. B. durch die Trockenheit der Haut vermindern. Aufgrund zunehmender Trockenheit der Haut wächst der Widerstand.

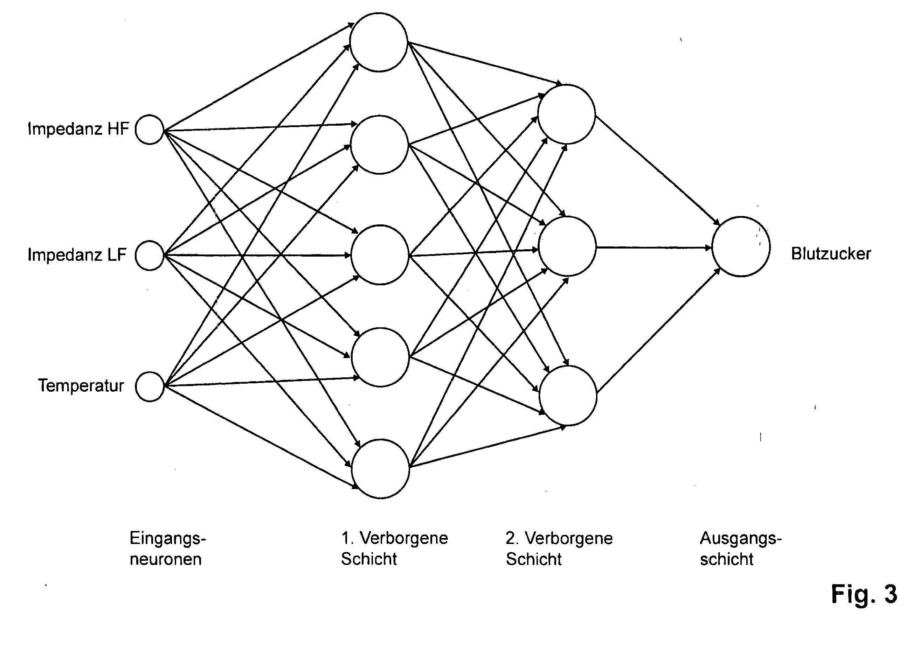

Ein weiteres Merkmal der Erfindung zeichnet sich dadurch aus, dass das künstliche neuronale Netzwerk als Netzwerk mit neun Netzen und mehreren Schichten ausgebildet ist (Fig. 2, Fig. 3). Ein künstliches neuronales Netz besteht aus Neuronen, die in Schichten angeordnet und durch Synapsen untereinander verbunden sind. Jedes neuronale Netz besitzt eine bestimmte Anzahl von Eingangsneuronen sowie eine Ausgangsschicht, die die Ausgangs-neuronen enthält. Weiterhin besitzt ein künstliches neuronales Netz üblicherweise mindestens eine verborgene Schicht, die die verborgenen Neuronen enthält. Über die Eingangsneuronen werden die vorhandenen Daten aufgenommen und in das neuronale Netz gegeben. Die Daten durchlaufen die Neuronen aller Schichten und werden dabei bearbeitet. Über die Ausgangsneuronen werden schließlich die berechneten Ergebnisse ausgegeben. Im Einzelnen erfolgt die Transformation der Daten bei der Weitergabe an die verschiedenen Schichten des neuronalen Netzes hierbei derart, dass ein Neuron Daten empfängt, und zwar entweder als Eingabedaten in das neuronale Netz oder in bearbeiteter Form von Neuronen der vorhergehenden Schicht. Die Daten werden gewichtet und anschließend addiert. Von der Summe wird ein Schwellenwert abgezogen und aus dem daraus resultierenden Ergebnis wird mit Hilfe der Transferfunktion der Wert berechnet, den das Neuron weitergibt

Bei der Erfindung wird vorteilhaft die Tan-Sigmoid Funktion

$$T(n) = 2/(1+e^{(-2n)}) -1$$

als Transferfunktion in den Neuronen der verborgenen Schichten genutzt. In den Eingangsneuronen sowie in den Neuronen der Ausgangsschicht wird die Identitätsfunktion

$$T(n) = 2/(1+e^{(-2n)}) -1$$

genutzt.

Es wird darauf hingewiesen, dass auch der Einsatz anderer Transferfunktionen möglich ist. Hierbei wird die Tan-Sigmoid Funktion in den Neuronen der verborgenen Schichten benutzt. In den Eingangsneuronen sowie in den Neuronen der Ausgangsschicht wird die Identitätsfunktion $T(n) = n$ als Transferfunktion genutzt.

Anhand der Zeichnung, Fig. 2, wird die Funktionsweise eines einzelnen Neurons näher erläutert.

Wie bereits an anderer Stelle erläutert, enthalten künstliche neuronale Netze mindestens eine Ausgangsschicht, vorteilhaft jedoch mindestens eine verborgene Schicht und eine Ausgangsschicht. Bei den künstlichen neuronalen Netzen gemäß der Erfindung sind jedoch zwei verborgene Schichten und eine Ausgangsschicht vorgesehen (Fig. 3), wobei insbesondere vorteilhaft bei der Erfindung die erste verborgene Schicht fünf Neuronen enthält, und die zweite dahinter angeordnete verborgene Schicht drei Neuronen und die Ausgangsschicht wieder ein Neuron, wie sich dies aus Figur 3 ergibt. Grundsätzlich ist die Anzahl der Neuronen in einem neuronalen Netz allerdings nicht beschränkt.

Es wurde ebenfalls bereits an anderer Stelle darauf hingewiesen, dass bei dem Verfahren durch die Verwendung eines künstlichen neuronalen Netzwerkes das Ergebnis mit der Anzahl der durchgeführten Messungen immer genauer wird. Das heißt, das neuronale Netz ist selbstständig lernfähig. Für die Genauigkeit der zu berechnenden Ergebnisse, und hier insbesondere des Blutzuckergehalts, ist die Wertebelegung der Gewichte und der Schwellenwerte entscheidend. Die Schwellenwerte und Gewichte innerhalb des neuronalen Netzes werden zunächst mit zufällig gewählten beliebigen Werten belegt. Zum Training werden dann dem Netz Daten, also Impedanzen und Temperaturwerte und die dazugehörigen Ergebnisse (Blutzuckergehalt) präsentiert. Das Netz berechnet aus den gegebenen Daten einen Wert, der mit dem bekannten Wert, hier dem invasiv gemessenen Blutzuckerwert, verglichen wird. Aufgrund der sich hierbei ergebenden Abweichungen werden die Schwellenwerte und Gewichte im Netz korrigiert. Dies geschieht durch numerische Verfahren, wie z. B. durch den Levenberg-Marquardt-Algorithmus. Dieser Vorgang des Lernens wird für ein neuronales Netz so lange wiederholt, bis die Abweichung einen gewünschten Wert nicht überschreitet.

Ausgehend von zufälligen Startwerten für die Wichtungsfaktoren in den neuronalen Netzen wird im Einzelnen zur Kalibrierung bzw. zum Anlernen oder Trainieren der einzelnen neuronalen Netze des Netzwerkes dem jeweiligen neuronalen Netz mindestens jeweils eine zu einem Zeitpunkt gemessene LF- und HF-Impedanz sowie vorteilhaft mindestens ein zum gleichen Zeitpunkt ermittelter Temperaturmesswert sowie mindestens ein in dem im Wesentlichen gleichen Zeitraum invasiv ermittelter Blutzuckerwert eingegeben, wobei der anhand der LF- und HF-Impedanzen und der Temperatur durch das neuronale Netzwerk ermittelte Blutzuckerwert mit dem invasiv gemessenen Blutzuckerwert verglichen wird, und mittels des Levenberg-Marquardt-Algorithmus die Abweichung reduziert wird. Zur Reduzierung der Abweichung zwischen dem gemessenen und dem durch das neuronale Netz rechnerisch ermittelten Blutzuckerwertes werden die in dem neuronalen Netz eingesetzten Gewichte und Schwellenwerte mit Hilfe des Levenberg-Marquardt-Algorithmus derart korrigiert, dass nach mehrmaligem Kalibrierungsvorgang die Abweichung minimiert ist. Mit anderen Worten bedeutet dies, dass nach jedem Berechnungsdurchgang, also nach jedem durch das neuronale Netz berechneten Blutzuckerwert dieser Wert mit dem tatsächlichen invasiv gemessenen Blutzuckerwert verglichen wird, die sich hieraus ergebende Abweichung mit Hilfe des Levenberg-Marquardt-Algorithmus zu einer Änderung der Gewichte und Schwellenwerte führt, mit der Folge, dass bei einem nachfolgenden Berechnungsvorgang in dem künstlichen neuronalen Netz die Abweichung des durch das Netzwerk rechnerisch ermittelten Blutzuckerwertes vom tatsächlichen invasiv gemessenen Blutzuckerwert geringer wird. Hieraus folgt unmittelbar, dass dann, wenn die Abweichung nicht nur von einem invasiv gemessenen Blutzuckerwert Berücksichtigung bei der Indizierung der Schwellenwerte und Gewichte findet, sondern eine Mehrzahl von tatsächlich gemessenen Blutzuckerwerten Eingang in das Verfahren findet, das neuronale Netz umso genauer in der Lage ist, den Blutzuckerwert zu berechnen. An dieser Stelle wird darauf hingewiesen, dass die invasiv ermittelten Blutzuckerwerte nicht unbedingt solche sind, die mit Hilfe von Blutzuckermessstationen ermittelt werden, sondern auch solche sein können, die in Kliniken chemisch gemessen werden und somit genauer sind. Es kann sein, dass während des Betriebes in bestimmten zeitlichen Abständen die Kalibrierung wiederholt werden muss.

Zur weiteren Erhöhung der Genauigkeit erfolgt die Berechnung des Blutzuckergehalts durch das neuronale Netzwerk nicht nur durch ein neuronales Netz, sondern durch eine Mehrzahl neuronaler Netze, insbesondere neun neuronaler Netze. Dies geschieht im Einzelnen durch eine Mittelwertbildung der Ausgabewerte, d. h. der berechneten Blutzuckerwerte der einzelnen Netze, und hier der insbesondere sieben Netze, deren Ausgabewerte die geringste Abweichung zu den invasiv gemessenen Werten aufweisen. Bevor allerdings die gemessenen Werte, d. h. die Impedanzen und die Temperatur, in die Netze gegeben werden, werden sie zunächst aufbereitet. Das heißt, die Impedanz- und Temperaturwerte werden mit Hilfe verschiedener Filter, z. B. dem Finite Impulse Response Filter 16. Ordnung oder auch dem Median-Filter 8. Ordnung geglättet, um eventuelle Messfehler auszugleichen. Zu invasiv ermittelten Blutzuckermesswerten werden mit Hilfe von Spline-Interpolation approximative Zwischenwerte berechnet. Dies vor folgendem Hintergrund: Zum Training benötigt ein künstliches neuronales Netz zu den zu einem bestimmten Zeitpunkt gemessenen LF-, HF-Impedanzen und Temperaturwerten einen Glukosewert desselben Zeitpunktes. Die Messung der LF-, HF-Impedanzen und Temperaturwerte findet kontinuierlich über einen bestimmten Zeitraum statt, woraus jeweils ein Wert pro Minute resultiert. Der Glukosewert wird jedoch nur zu bestimmten Zeitpunkten in diesem Zeitraum invasiv gemessen (also nicht jede Minute). Zum Training benötigen die künstlichen neuronalen Netze aber zu allen vorhandenen LF-, HF-Impedanzen und Temperaturwerten einen entsprechenden Glukosewert. Deshalb werden die invasiv gemessenen Glukosewerte mit Hilfe einer Interpolationsfunktion miteinander verbunden. Dies geschieht durch Spline-Interpolation. Mit Hilfe der Interpolationsfunktion werden dann zu jeder LF-, HF-Impedanz und jedem Temperaturwert approximative Zwischenwerte des Glukosewertes berechnet. Anders ausgedrückt, wird durch die gegebenen Glukosewerte eine Kurve gelegt. An dieser Kurve wird zu jeder LF-, HF-Impedanz und jedem Temperaturwert ein approximativer Glukosewert abgelesen.

Betrachtet werden allerdings nicht nur die aktuellen Werte für die HF- und LF-Impedanz sowie die aktuelle Temperatur, sondern auch die mindestens zwei vorhergehenden entsprechenden Werte. Solche Werte fließen, wenn sie relevant sind, automatisch über zusätzliche Eingangsneuronen in die Berechnung ein. Das heißt, die genaue Zahl der Eingangsneuronen wird durch eine Hauptkomponentenanalyse der aktuell sowie z. B. vor einer Minute und z. B. vor zwei Minuten gemessene Impedanz- und Temperaturwerte bestimmt. Die Hauptkomponentenanalyse wird über alle neun der zu den drei Zeitpunkten ermittelten Messwerte durchgeführt. Durch die Hauptkomponentenanalyse ist es möglich, die Dimension der Daten mit minimalem Informationsverlust zu reduzieren, was dadurch geschieht, dass eine Menge korrelierter Variablen in eine Menge unkorrelierter Variablen transformiert wird. Zur Kalibrierung werden diese Daten normalisiert, d. h. sie werden so angepasst, dass ihre Mittel und Standardabweichungen 0 bzw. 1 betragen. Mit den normalisierten Daten wird eine Hauptkomponentenanalyse durchgeführt, wobei die daraus resultierenden Daten als Eingangsneuronen für das künstliche neuronale Netz dienen. Die gemessenen und interpolierten Blutzuckerwerte werden ebenfalls normalisiert. Die Transformationsdaten werden gespeichert. Die Netze werden mit den aufbereiteten Daten trainiert. Nach Abschluss des Trainings werden die Ausgabewerte der Netze sowie die Eingangsdaten de-normalisiert, so dass ihre Mittelwerte und Standardabweichungen wieder die ursprünglichen Werte annehmen.

Im Berechnungsmodus werden die Daten mit Hilfe der während des Trainings berechneten Mittelwerte und Standardabweichungen normalisiert und die Komponenten der Eingangsdaten werden mit Hilfe der gespeicherten Transformationsmatrix reduziert. Nachdem die Netze die Berechnungen durchgeführt haben, werden die Ausgabewerte denormalisiert, so dass der Blutzuckerwert in der gewünschten Form ausgegeben wird. Alsdann findet die bereits oben beschriebene Mittelwertbildung sämtlicher Ausgabewerte einer bestimmten Anzahl der besten Netze statt. Dieser Mittelwert wird dann als augenblicklicher Blutzuckerwert angegeben.

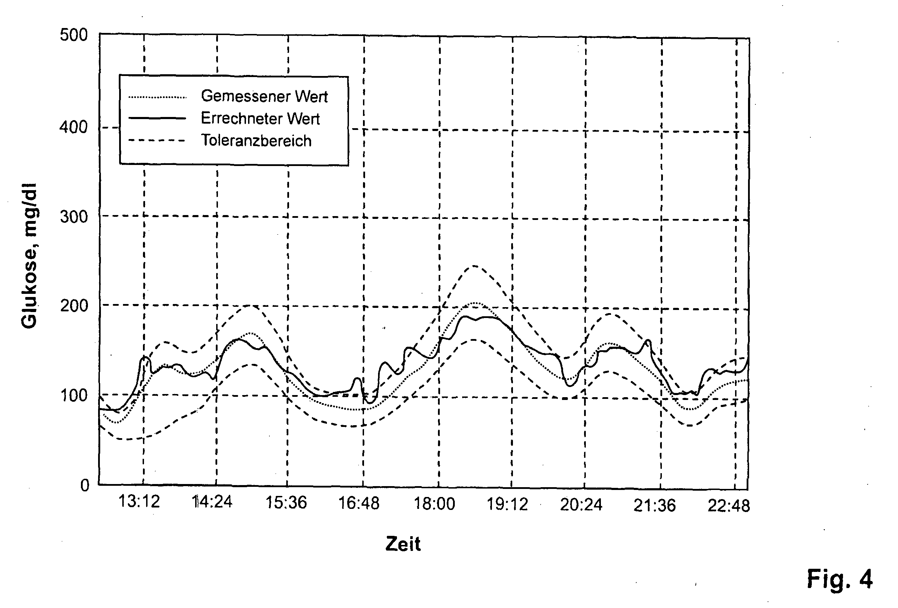

Aus Fig. 4 ist ein beispielhaftes Messergebnis erkennbar. Es ist ersichtlich, dass sich die berechneten Blutzuckerwerte im Rahmen eines vorgegebenen Toleranzbereichs bewegen. Die Abweichung der berechneten Werte von den invasiv gemessenen Werten beträgt etwa zwischen 2 und 3 %.

Unter Zugrundelegung folgender Parameter werden die Kurven nach Fig. 4 ermittelt:

Der hoch- und der niederfrequente Strom betragen 800 mA; die Spannung beträgt jeweils 1 V. Die Frequenz $H_F$ beträgt 2,5 MHz. Die Frequenz $L_F$ beträgt 21 kHz. Invasiv wurde der Blutzuckerwert alle 15 Minuten bestimmt. Die Messung der Impedanzen und die rechnerische Ermittlung des Blutzuckerwertes erfolgte jede Minute. Die invasiv gemessenen Blutzuckerwerte werden interpoliert mit dem Ziel, jedem rechnerisch ermittelten Wert einen invasiv gemessenen bzw. interpolierten Wert zuzuordnen. Schwankungen im Kurvenverlauf sind u. a. bedingt durch folgende Faktoren: Nahrungsaufnahme und körperliche Aktivität. Die Oberflächentemperatur der Haut lag über den Zeitraum von etwa 12:30 Uhr bis 22:48 Uhr zwischen 24,73°C und 26,37°C.

## Patentansprüche

1. Verfahren zur nicht invasiven Bestimmung des Blutzuckergehaltes im Blut durch Messung der Impedanzen des Körpers des Patienten oder eines Teils des Körpers des Patienten,
   das
   mindestens folgende Schritte enthält:

   - Einbringen eines hoch- und eines niederfrequenten Stromes in den Körper eines Patienten, wobei Elektroden zur Einbringung des hoch- und des niederfrequenten Stroms beabstandet zueinander am Körper des Patienten angelegt sind; Erfassung der HF- und LF-Impedanzen durch eine Messeinrichtung und Eingabe der ermittelten HF- und LF-Impedanzen in ein kalibriertes neuronales Netzwerk;
   - Generierung einer Mehrzahl von neuronalen Netzen mit unterschiedlichen Startwerten zur genaueren Ermittlung der Impedanz zum Training des neuronalen Netzwerkes, wobei von der Mehrzahl der Netze eine Anzahl von Netzen mit den Ausgabewerten herangezogen wird, die zu denen der während des Kalibrierungsschrittes invasiv ermittelten Blutzuckerwerte die geringste Abweichung aufweisen;
   - Kalibrierung des künstlichen neuronalen Netzwerkes durch Eingabe mindestens jeweils einer über einen bestimmten Zeitraum gemessenen LF- und HF-Impedanz sowie gegebenenfalls mindestens jeweils eines Temperaturmesswertes in die neuronalen Netze sowie mindestens eines in dem Zeitraum invasiv ermittelten Blutzuckerwertes, wobei der anhand der HF-und LF-Impedanzen und gegebenenfalls der Temperatur durch das künstliche neuronale Netz ermittelte Blutzuckerwert mit dem invasiv gemessenen Blutzuckerwert verglichen und mittels eines numerischen Verfahrens, zum Beispiel mittels des Levenberg-Marquardt-Algorithmus, die Abweichung reduziert wird;
   - Ermittlung des Blutzuckergehalts durch Mittelwertbildung der Ausgabewerte der herangezogenen Netze;
   - Anzeige des durch das neuronale Netzwerk ermittelten Blutzuckergehalts auf einer Anzeige.

2. Verfahren nach Anspruch 1,
   **dadurch gekennzeichnet**,
   das die Körperoberflächentemperatur ermittelt wird und der Temperaturmesswert in das neuronale Netz eingegeben wird.

3. Verfahren nach einem der voranstehenden Ansprüche,
   **dadurch gekennzeichnet,**
   **dass** die Körperoberflächentemperatur am Körper des Patienten im Bereich der Elektroden gemessen wird.

4. Verfahren nach einem der voranstehenden Ansprüche,
   **dadurch gekennzeichnet,**
   **dass** die Körperoberflächentemperatur zum Zeitpunkt der Messung der Impedanz ermittelt wird.

5. Verfahren nach einem der voranstehenden Ansprüche,
   **dadurch gekennzeichnet,**

**dass** das künstliche neuronale Netz als neuronales Netz mit drei Schichten ausgebildet ist.

6. Verfahren nach einem der voranstehenden Ansprüche,
   **dadurch gekennzeichnet,**
   **dass** die Temperatur als Startwert der Mehrzahl der neuronalen Netze neben der Impedanz angegeben wird.

7. Verfahren nach einem der voranstehenden Ansprüche,
   **dadurch gekennzeichnet,**
   **dass** in das künstliche neuronale Netz eingegebene Messwerte durch Filter geglättet werden.

8. Verfahren nach einem der voranstehenden Ansprüche,
   **dadurch gekennzeichnet,**
   **dass** zur Reduzierung der Abweichung zwischen dem gemessenen und dem durch das künstliche neuronale Netzwerk ermittelten Blutzuckerwert die in dem künstlichen neuronalen Netzwerk eingesetzten Gewichte und Schwellenwerte mit Hilfe des Levenberg-Marquardt-Algorithmus derart korrigiert werden, dass nach mehrmaligem Kalibrierungsdurchgang die Abweichung minimiert wird.

9. Einrichtung zur nicht invasiven Bestimmung des Blutzuckergehalts im Blut durch Messung der Impedanzen des Körpers des Patienten oder eines Teils des Körpers des Patienten, umfassend zwei beabstandet zueinander am Körper des Patienten anbringbare Elektroden zur Einbringung eines nieder- und eines hochfrequenten Stroms in den Körpers des Patienten,
   weiterhin umfassend eine Messeinrichtung zur Erfassung der Impedanzen sowie eine Rechnereinheit mit einem Mikroprozessor zur Verarbeitung der ermittelten HF- und LF-Impedanzen in einem in dem Mikroprozessor installierten kalibrierten neuronalen Netzwerk, wobei zum Training des neuronalen Netzwerkes eine Mehrzahl von neuronalen Netzen mit unterschiedlichen Startwerten zur genaueren Ermittlungen des Blutzuckerwertes aus gemessenen Impedanzen generiert wird, wobei von der Mehrzahl der Netze eine Anzahl von Netzen mit den Ausgabewerten herangezogen wird, die zu denen während des Kalibrierungsschrittes invasiv ermittelten Blutzuckerwerten die geringste Abweichung aufweisen,
   wobei die Kalibrierung des künstlichen neuronalen Netzwerkes durch Eingabe mindestens jeweils einer über einen bestimmten Zeitraum gemessenen LF- und HF-Impedanz sowie gegebenenfalls mindestens jeweils eines Temperaturmesswerkes in die neuronalen Netze sowie mindestens eines in dem Zeitraum invasiv ermittelten Blutzuckerwertes erfolgt, wobei der anhand der HF-und LF-Impedanzen und gegebenenfalls der Temperatur durch das künstliche neuronale Netz ermittelte Blutzuckerwert mit dem invasiv gemessenen Blutzuckerwert verglichen und mittels eines numerischen Verfahrens, zum Beispiel mittels des Levenberg-Marquardt-Algorithmus, die Abweichung reduziert wird, wobei der Blutzuckergehalt durch Mittelwertbildung der Ausgabewerte der herangezogenen neuronalen Netze ermittelt wird, weiterhin umfassend eine Anzeigeeinrichtung zur Anzeige des durch das neuronale Netzwerk ermittelten Blutzuckergehalts.

10. Einrichtung nach Anspruch 9,
    **dadurch gekennzeichnet,**
    **dass** jeweils ein Elektrodenpaar für die Einbringung des niederfrequenten und des hochfrequenten Stroms eingesetzt werden.

**Claims**

1. A method for noninvasive determination of blood glucose levels in the blood by measuring the impedances of the body of the patient or of a part of the body of the patient, which includes at least the following steps:

   - introducing a high and a low frequency current into the body of the patient, wherein the electrodes for introducing the high and the low frequency current are applied at a distance from each other onto the body of the patient;
   - capturing the HF and LF impedances by way of a measuring device and inputting the determined HF and LF impedances into a calibrated neural network;
   - generating a plurality of neural networks with different initial values for a more precise determination of the impedance in order to train the neural network, wherein among the plurality of networks a number of networks with output values are drawn upon, which have the slightest deviation from the blood glucose levels invasively determined during the calibration step;
   - calibrating the artificial neural networks by inputting at least respectively one LF and HF impedance measured over a determined time period, and, if applicable, respectively at least one temperature value into the neural networks as well as at least one blood glucose value invasively determined in that time period, wherein the blood glucose value calculated by the artificial neural network based on the HF and LF impedances and, if applicable, on the temperature is compared with the invasively measured blood glucose value and the deviation is reduced by means of a numerical method, for example by means of the Levenberg-Marquardt algorithm;
- calculating the blood glucose level by averaging the output values of the used networks;
- displaying the blood glucose level calculated by the neural network on a display.

**2.** The method according to claim 1,
   **characterized in that**
   the body surface temperature is determined and the temperature value is input into the neural network.

**3.** The method according to one of the afore-mentioned claims,
   **characterized in that**
   the body surface temperature is measured on the body of the patient in the area of the electrodes.

**4.** The method according to one of the afore-mentioned claims,
   **characterized in that**
   the body surface temperature is determined at the moment of measurement of the impedance.

**5.** The method according to one of the afore-mentioned claims,
   **characterized in that**
   the artificial neural network is formed as a neural network with three layers.

**6.** The method according to one of the afore-mentioned claims,
   **characterized in that**
   the temperature is specified as an initial value of the plurality of neural networks, in addition to the impedance.

**7.** The method according to one of the afore-mentioned claims,
   **characterized in that**
   the measurement values input into the artificial neural network are smoothed by filters.

**8.** The method according to one of the afore-mentioned claims,
   **characterized in that**
   in order to reduce the deviation between the measured blood glucose value and the blood glucose value calculated by the artificial neural network, the weighting and threshold values used in the artificial neural network are corrected by means of the Levenberg-Marquardt algorithm in such a manner that the deviation is minimized after a repeated calibration cycle.

**9.** A device for noninvasive determination of the blood glucose levels in the blood by measuring the impedances of the body of the patient or of a part of the body of the patient, comprising two electrodes, which are attachable at a distance from each other to the body of the patient for introducing a low and a high frequency current into the body of the patient,
   further comprising a measuring device for capturing the impedances as well as a computer unit with a micro-processor for processing the determined HF and LF impedances in a calibrated neural network installed in the micro-processor, wherein in order to train the neural network, a plurality of neural networks with different initial values is generated for a more precise calculation of the blood glucose value based on the measured impedances, wherein among the plurality of networks a number of networks with the output values are drawn upon, which show the slightest deviation from the blood glucose values invasively determined during the calibration step,
   wherein the calibration of the artificial neural network occurs by inputting at least respectively one LF and HF impedance measured over a determined time period and, if applicable, at least respectively one temperature value into the neural networks, as well as at least one blood glucose value invasively determined in that time period, wherein the blood glucose value calculated by the artificial neural network based on the HF and LF impedances and, if applicable, the temperature is compared to the invasively measured blood glucose value and the deviation is reduced by means of a numerical method, for example by means of the Levenberg-Marquardt algorithm, wherein the blood glucose level is calculated by averaging the output values of the used neural networks,
   further comprising a display device for displaying the blood glucose level calculated by the neural network.

**10.** The device according to claim 9,
**characterized in that**
respectively one pair of electrodes is used for introducing the low-frequency and high-frequency current.

**Revendications**

**1.** Procédé de détection non invasive de la teneur en sucre du sang par la mesure des impédances du corps du patient ou d'une partie du corps du patient, comprenant au moins les étapes suivantes :

• l'amenée d'un courant de haute fréquence et de basse fréquence dans le corps du patient, au cours de laquelle des électrodes pour l'amenée de la haute fréquence et de la basse fréquence dans le corps du patient sont distantes l'une par rapport à l'autre ;
• la détection des impédances de HF et de BF au moyen d'une installation de mesure et l'enregistrement des impédances détectées dans un réseau neuronal calibré ;
• la génération d'une pluralité de réseaux neuronaux avec différentes valeurs initiales pour la détermination plus précise de l'impédance en vue de l'entraînement des réseaux neuronaux, dans laquelle, de la pluralité de réseaux, une série de réseaux avec les valeurs déterminées sont extraits, ces valeurs présentant une dérive minimale par rapport aux valeurs de taux de sucre dans le sang relevées invasivement au cours de l'étape de calibrage,
• le calibrage des réseaux neuronaux artificiels par l'introduction d'au moins une impédance mesurée HF et BF respectivement sur un intervalle de temps déterminé ainsi qu'au moins une valeur du taux de sucre dans le sang déterminée invasivement, dans laquelle la teneur en sucre dans le sang déterminée au moyen de la mesure d'impédance HF et BF et, le cas échéant de la température, par le réseau neuronal artificiel est comparée à la teneur en sucre dans le sang mesurée invasivement et est réduit, par exemple au moyen d'un algorithme de Levenberg-Marquardt, réduisant la différence ;
• la détermination de la teneur en sucre dans le sang par la détermination d'une valeur moyenne des réseaux considérés ;
• l'affichage de la teneur en sucre dans le sang déterminée par le réseau neuronal, sur un afficheur.

**2.** Procédé selon la revendication 1,
**caractérisé en ce que,**
la température de surface du corps est déterminée et est introduite comme valeur de température dans le réseau neuronal.

**3.** Procédé selon l'une des revendications précédentes,
**caractérisé en ce que,**
la température de surface du corps est mesurée sur le corps du patient dans la zone des électrodes.

**4.** Procédé selon l'une des revendications précédentes,
**caractérisé en ce que,**
la température de surface du corps est déterminée au moment de la mesure des impédances.

**5.** Procédé selon l'une des revendications précédentes,
**caractérisé en ce que,**
le réseau neuronal artificiel est constitué d'un réseau neuronal à trois couches.

**6.** Procédé selon l'une des revendications précédentes,
**caractérisé en ce que,**
la température est donnée comme valeur initiale de la pluralité des réseaux neuronaux en plus de l'impédance.

**7.** Procédé selon l'une des revendications précédentes,
**caractérisé en ce que**,
les valeurs introduites dans le réseau neuronal artificiel sont lissées par filtrage.

**8.** Procédé selon l'une des revendications précédentes,
**caractérisé en ce que**,
la réduction de l'écart entre les teneurs en sucre dans le sang mesurée et déterminée par le réseau neuronal artificiel et les poids et les valeurs seuils introduits dans le réseau neuronal artificiel est corrigée au moyen de l'algorithme de Levensberg-Marquardt, de telle manière que plusieurs passages par la calibration minimise l'écart.

9.  Dispositif pour la détection non invasive de la teneur en sucre du sang par la mesure des impédances du corps du patient ou d'une partie du corps du patient, comprenant deux électrodes écartées pouvant être positionnées sur le corps du patient pour amener un courant de haute fréquence et un courant de basse fréquence,
    comportant par ailleurs une installation de mesure pour la détermination des impédances ainsi qu'une unité de calcul comprenant un microprocesseur pour le traitement des impédances HF et BF déterminées, dans un réseau neuronal calibré installé dans le microprocesseur, dans lequel, en vue de l'apprentissage des réseaux neuronaux, une pluralité de réseaux neuronaux avec des valeurs initiales distinctes, sont utilisés pour générer une mesure plus précise de la teneur en sucre dans le sang à partir des impédances mesurées, dans lequel une série de réseaux est extraite avec leurs valeurs déterminées, de la pluralité de réseaux, qui sont comparées aux valeurs de la teneur en sucre dans le sang mesurées de façon invasive pendant la phase de calibrage, présentant le minimum de dérive, dans lequel le calibrage des réseaux neuronaux artificiels s'effectue par l'introduction d'au moins une impédance mesurée HF et BF respectivement sur un intervalle de temps déterminé ainsi qu'au moins une valeur de température respectivement dans les réseaux neuronaux ainsi qu'au moins une valeur du taux de sucre dans le sang déterminé invasivement, dans laquelle la teneur en sucre dans le sang déterminée au moyen de la mesure d'impédance HF et BF et, le cas échéant de la température par le réseau neuronal artificiel est comparée à la teneur en sucre dans le sang mesurée invasivement et est réduit, par exemple au moyen d'un algorithme de Levenberg-Marquardt, dans lequel la teneur en sucre dans le sang est déterminée par une moyenne des valeurs obtenues dans les réseaux neuronaux considérés,
    comportant en outre une installation d'affichage de la teneur en sucre dans le sang.

10. Dispositif selon la revendication 9,
    **caractérisé en ce que**,
    du courant électrique est introduit respectivement dans une paire d'électrodes pour l'application de courant de haute fréquence et de basse fréquence.

**Fig. 1**

w(i): Gewichte

x(i): Daten

$\theta$: Schwellenwert

T: Transferfunktion

**Fig. 2**

Fig. 3

Fig. 4

EP 2 196 140 B1

**IN DER BESCHREIBUNG AUFGEFÜHRTE DOKUMENTE**

**In der Beschreibung aufgeführte Patentdokumente**

- US 6841389 B2
- WO 02069798 A1
- EP 13092711 A
- WO 2008141306 A2